(19) Europäisches Patentamt
European Patent Office
Office européen des brevets

(11) Veröffentlichungsnummer : **0 140 210**
**B1**

(12) # EUROPÄISCHE PATENTSCHRIFT

(45) Veröffentlichungstag der Patentschrift : **27.07.88**

(51) Int. Cl.⁴ : **C 07 C 69/593**, C 07 C 67/347

(21) Anmeldenummer : **84111940.7**

(22) Anmeldetag : **05.10.84**

(54) **Verfahren zur katalytischen Dimerisation von Acrylsäurederivaten und Verwendung der erhaltenen Dimeren.**

(30) Priorität : **08.10.83 DE 3336691**

(43) Veröffentlichungstag der Anmeldung : **08.05.85 Patentblatt 85/19**

(45) Bekanntmachung des Hinweises auf die Patenterteilung : **27.07.88 Patentblatt 88/30**

(84) Benannte Vertragsstaaten : **AT BE CH DE FR GB IT LI LU NL**

(56) Entgegenhaltungen :
**DE-A- 1 917 884**
**FR-A- 2 079 319**
**FR-A- 2 524 341**
**US-A- 3 853 961**
**US-A- 4 144 257**

(73) Patentinhaber : **Studiengesellschaft Kohle mbH**
**Kaiser-Wilhelm-Platz 1**
**D-4330 Mülheim/Ruhr (DE)**

(72) Erfinder : **Wilke, Günther, Prof. Dr.**
**Kaiser-Wilhelm-Platz 1**
**D-4330 Mülheim/Ruhr (DE)**
Erfinder : **Sperling, Karin, Dr.**
**Dimbeck 39a**
**D-4330 Mülheim/Ruhr (DE)**
Erfinder : **Stehling, Ludwig**
**Dimbeck 46**
**D-4330 Mülheim/Ruhr (DE)**

(74) Vertreter : **von Kreisler, Alek, Dipl.-Chem. et al**
**Deichmannhaus am Hauptbahnhof**
**D-5000 Köln 1 (DE)**

**EP 0 140 210 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur katalytischen Dimerisation von Derivaten der Acrylsäure, d. h. zur Herstellung on Dicarbonsäurederivaten, die noch eine Doppelbindung enthalten. Die erfindungsgemäß herstellbaren Produkte sind wertvolle Monomere für die Herstellung von Polymeren. Nach dem beanspruchten Verfahren lassen sich insbesondere lineare Dimere in hoher Selektivität herstellen.

Es ist bekannt, daß tertiäre Phosphane, Phosphite und bestimmte tertiäre Amine Acrylsäurederivate zu dimerisieren vermögen, wobei jedoch bevorzugt verzweigte Dimere, z. B. aus Acrylsäuremethylester α-Methylenglutarsäuremethylester, entstehen.

Es ist ferner bekannt, daß Palladium(II)- oder Rhodium(III)-Verbindungen Acrylsäuremethylester in der Hitze und unter Druck dimerisieren, wobei jedoch die Ausbeuten unter 70 % liegen und die Selektivität für lineare Dimere sehr klein ist.

Es ist ebenfalls bekannt, daß Rhodium(III)-Verbindungen Acrylamid in siedendem Ethanol sehr langsam und mit schlechten Ausbeuten dimerisieren.

Darüber hinaus ist bekannt, daß Acrylnitril und Acrylsäureester unter dem Einfluß von Rutheniumverbindungen unter $H_2$-Druck in der Hitze in Dihydrodimere und zahlreiche Nebenprodukte übergehen.

Schließlich ist bekannt, daß lösliche Palladium(II)-Komplexe in Kombination mit Chinonen ungesättigte Carbonsäurederivate der allgemeinen Formel $CH_2 = CH—COOR$ bei Temperaturen von 0 °C bis zum Siedepunkt der Substrate dimerisieren.

Alle bekannten Verfahren sind unter technischen Gesichtspunkten nur bedingt durchführbar, da entweder nur verzweigte Produkte entstehen (Phosphane usw.) oder aber Katalysatoren auf der Basis extrem teurer Metalle (Pd, Rh, Ru) eingesetzt werden müssen, die, was besonders nachteilig ist, nur niedrige Cyclenzahlen (maximal ca. 45 Dimerisationsschritte pro Katalysatormolekül bzw. pro Metallatom) ergeben.

Mit den erfindungsgemäßen Verfahren werden die genannten Nachteile der bisher bekannten Verfahren überwunden, indem mit Katalysatoren auf der Basis eines preiswerten Übergangsmetalls (Ni) unter milden Bedingungen hohe Selektivitäten bezüglich der linearen Dimeren und zugleich hohe Cyclenzahlen (> 300 Mol Dimere pro Mol Katalysator) erzielt werden.

Gegenstand der Erfindung ist somit ein Verfahren zur Dimerisation von Derivaten der Acrylsäure in homogener Phase, das dadurch gekennzeichnet ist, daß man Verbindungen der allgemeinen Formel $H_2C = CR^1—COOR^2$, in der $R^1$ für H oder lineare Alkylgruppen mit 1 bis 3 C-Atomen und $R_2$ für lineare Alkylgruppen mit 1 bis 3 C-Atomen stehen, in einem Lösungsmittel bei Temperaturen von —50 °C bis + 50 °C in Gegenwart von Nickelverbindungen als Katalysatoren umsetzt, die komplexe Anionen X in einem Molverhältnis Ni : X = 1 : 1 und organische oder Hydrid-Liganden enthalten und mit Phosphanen der allgemeinen Formel $PR_2R'$ modifiziert werden, in der R und R' gleich oder verschieden sein können und für Alkylreste mit 1 bis 8 C-Atomen oder Phenylreste stehen, sowie die Verwendung der bei der Dimerisation entstehenden Dimeren.

Die Katalysatoren, die in dem erfindungsgemäßen Verfahren verwendet werden, sind lösliche Nickelverbindungen, die komplexe Anionen X enthalten. Als komplexe Anionen X kommen insbesondere Fluor enthaltende Anionen, wie z. B. $BF_4^-$, $PF_6^-$ oder $SbF_6^-$, in Frage. In den als Katalysator verwendeten Nickelverbindungen liegen diese Fluorokomplexe im Mol-Verhältnis Ni : komplexem Anion X wie 1 : 1 vor.

Die löslichen Nickelverbindungen enthalten darüber hinaus Alkyl-, Cycloalkyl-, Aralkyl, Allyl-, Cycloalkenyl- oder Arylgruppen oder ein Hydridion als Liganden. Bevorzugte Liganden in diesem Sinne sind die $\eta^3$-Allylgruppe, die $\eta^3$-Cyclooctenylgruppe oder gegebenenfalls alkylsubstituierte Phenylgruppen wie der Mesityl-Rest.

Diese löslichen Katalysatorsysteme enthalten weiterhin als modifizierende Liganden Phosphane der allgemeinen Formel $PR_2R'$, in der R und R' gleich oder verschieden sein können und für lineare Alkylreste mit 1 bis 8 C-Atomen oder Phenylreste stehen. Bevorzugt werden Tri-n-alkylphosphane, insbesondere Trimethylphosphan, verwendet. Das Molverhältnis Ni : Phosphan beträgt 1 : 0,5 bis 1,5, bevorzugt 1 : 1.

Die Katalysatoren des erfindungsgemäßen Verfahrens sind auf mehreren Wegen zugänglich. So können Katalysatoren z. B. aus $\eta^3$-Allyl-nickelhalogeniden bzw. deren 1 : 1-Phosphanaddukten und den Silbersalzen der genannten komplexen Anionen hergestellt werden :

$$\eta^3\text{-}C_3H_5Ni(PMe_3)Cl + AgBF_4 \longrightarrow \eta^3\text{-}C_3H_5Ni(PMe_3)BF_4 + AgCl \qquad (1)$$

Eine weitere Möglichkeit zur Katalysatorherstellung besteht in der Umsetzung von Bis ($\eta^3$-allyl) nickelverbindungen mit den Säuren der genannten komplexen Anionen X, z. B.

$$(\eta^3\text{-}C_3H_5)_2Ni + HBF_4 \longrightarrow \eta^3\text{-}C_3H_5NiBF_4 + C_3H_6 \qquad (2)$$

$$\eta^3\text{-}C_3H_5NiBF_4 \xrightarrow{+PMe_3} \eta^3\text{-}C_3H_5Ni(PMe_3)BF_4$$

Zu wirksamen Katalysatoren gelangt man auch durch eine Umsetzung von komplexen Anionen im Sinne einer oxidativen Addition z. B. von Bis (cyclooctadien) nickel (O) ($COD_2Ni$) und HX.

$$[COD]_2Ni + HBF_4 \xrightarrow[-COD]{+PMe_3} C_8H_{13}Ni(PMe_3)BF_4 \quad (3)$$

Die auf diesem Wege entstehende Komplexverbindung enthält neben dem komplexen Anion eine $C_8H_{13}$-Gruppe.

Zu Komplexen, die analog Arylgruppen enthalten, gelangt man durch Umsetzung entsprechender Nickeldiaryle mit HX z. B.

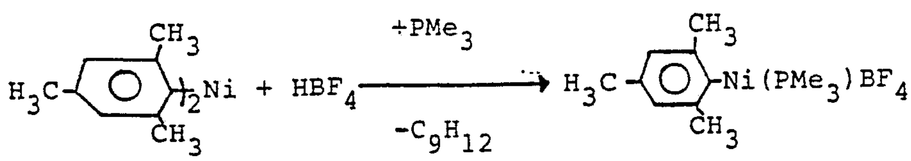


(4)

Es wird angenommen, daß im Zuge der katalytischen Dimerisationen die in den Ausgangskomplexen enthaltenen organischen Reste abgelöst werden, so daß als eingentlich wirksame Katalysatorspezies ein $HNi(PMe_3)BF_4$ gebildet wird, die während des Katalysecyclus durch Addition der HNi-Gruppe an ein ungesättigtes Monomeres in eine R-Ni-Gruppe z. B. eine $MeOCOCH_2$-$CH_2$-Ni-Gruppe übergeht, die unter Addition der Ni-C-Bindung an ein weiteres Monomeres und β-H-Eliminierung sowie Abspaltung des Dimeren wiederum das Hydrid $HNi(PMe_3)BF_4$ zurückbildet, das unmittelbar in den nächsten Cyclus eingeht.

Mit Hilfe der beispielhaft genannten Katalysatoren lassen sich z. B. Ester der Acrylsäure der allgemeinen Formel $CH_2 = CH\text{-}CO_2R^2$, wobei $R^2$ für Alkylreste mit 1 bis 3 C-Atomen steht, bevorzugt für Alkylreste mit 1 bzw. 2 C-Atomen steht, mit hoher Selektivität (> 90 %) in lineare Dimere mit einer Doppelbindung in 2- bzw. 3-Stellung umwandeln. Als Nebenprodukte fallen geringe Mengen verzweigter monoungesättigter Dicarbonsäureester an. Höhere Oligomere bzw. Polymere entstehen praktisch nicht. Analog gelingt erfindungsgmäß die Dimerisation der Ester der Methacrylsäure ($R^1 = CH_3$, $R^2 = CH_3$, $C_2H_5$) mit hoher Spezifität.

Die Dimerisation wird bevorzugt in polaren Lösungsmitteln wie z. B. Methylenchlorid oder Chlorbenzol ausgeführt, wobei mit steigender Verdünnung des Katalysators die erreichbaren Cyclenzahlen ansteigen.

Die Dimerisation kann in einem Temperaturbereich von —50 °C bis + 50 °C ausgeführt werden, bevorzugt jedoch bei Temperaturen von —20 °C bis + 20 °C.

Die erfindungsgemäß in hoher Selektivität herstellbaren überwiegend linearen Dicarbonsäureester mit einer Doppelbindung in 2- bzw. 3-Stellung können als wertvolle Monomere für die Herstellung von Polyamiden bzw. Polyestern eingesetzt werden. Die im Zuge der Polykondensationsreaktionen entstehenden Polymeren können aufgrund der vorhandenen Doppelbindungen vernetzt werden und ermöglichen dadurch die Herstellung von Polymeren für viele Anwendungsbereiche. Andererseits eröffnet das erfindungsgemäße Verfahren einen neuartigen, wirtschaftlich interessanten Zugang zu Adipinsäure bzw. deren Estern, da sich die linearen Dimeren der Acrylsäure katalytisch glatt hydrieren lassen.

Die Erfindung wird anhand der nachfolgenden Beispiele erläutert. Sämtliche in den Beispielen beschriebenen Versuche wurden unter Argon als Schutzgas durchgeführt.

## Beispiel 1

0,29 g (2,06 mmol) $(\eta^3\text{-}C_3H_5)_2Ni$ wurden in 50 ml $CH_2Cl_2$ zusammen mit 0,19 ml = 0,14 g (1,85 mmol) Trimethylphosphan ($PMe_3$) bei —78 °C mit 0,181 g (2,059 mmol) $HBF_4$ d. h. 0,28 ml einer 54 %igen etherischen Lösung umgesetzt. Nach 30 Minuten bei —78 °C ließ man diese Katalysatorlösung in eine auf 0 °C gekühlte Lösung von 192 g (2,4 Mol) Acrylsäuremethylester (AME) in 400 ml $CH_2Cl_2$ einfließen (Ni : AME = 1 : 1 082). Nach 46 Stdn. wurden alle flüchtigen Produkte im Vakuum abgezogen. Als Rückstand verblieb praktisch nur die eingesetzte Katalysatormenge neben Spuren nichtflüchtiger Verbindungen. Laut GC betrug der Umsatz 71 %, d. h. 136 g AME wurden dimerisiert. Das Destillat enthielt neben 56 g AME 122 g lineare und 14 g verzweigte monoungesättigte Diester. Die Selektivität bezogen auf lineare Diester betrug 90 %. Pro g-Atom Ni wurden demnach 692 Mol AME dimerisiert bzw. 346 Cyclen durchlaufen.

## Beispiel 2

Durchführung analog Beispiel 1, jedoch wurden in zeitlichen Intervallen Proben entnommen und der jeweilige Umsatz bestimmt :

| Probenentnahme nach h | Umsatz % | % Selektivität bezogen auf lineare Ester |
|---|---|---|
| 2 | 26 | 91 |
| 4 | 35 | 92 |
| 8 | 50 | 92,5 |
| 22 | 61 | 92 |
| 46 | 65 | 92 |
| 70 | 69 | 92 |

Pro mol Ni wurden 622 mol AME umgesetzt, d. h. 311 Cyclen durchlaufen.

## Beispiel 3

0,530 g (2,39 mmol) $C_{12}H_{18}Ni$, das entsprechend der nachfolgenden Reaktionsgleichung

CDT.Ni + 3 $C_4H_6$ 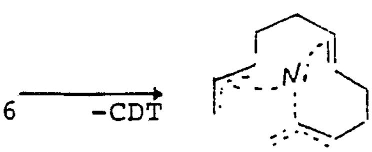

= $C_{12}H_{18}Ni$

dargestellt worden war, wurden in 50 ml $CH_2Cl_2$ zusammen mit 0,22 ml = 0,164 g (2,15 mmol) $PMe_3$ bei —78 °C mit 0,21 g (2,39 mmol) $HBF_4$, d. h. 0,33 ml einer 54 %igen etherischen Lösung umgesetzt und nach 30 Minuten in eine auf 0 °C gekühlte Lösung von 192 g (2,30 mol) AME in 450 ml $CH_2Cl_2$ eingetragen. (Ni : AME = 1 : 962). Wie in Beispiel 2 wurden Proben gaschromatographisch analysiert :

| Probenentnahme nach h | Umsatz % | % Selektivität bezogen auf lineare Diester |
|---|---|---|
| 2 | 7,8 | 92 |
| 4 | 15 | 92 |
| 8 | 30,5 | 92 |
| 20 | 50,5 | 92 |
| 46 | 64 | 92 |
| 70 | 69 | 91 |
| 94 | 71 | 91 |
| 118 | 78 | 91 |

Insgesamt wurden pro mol Ni 672 mol AME umgesetzt, d. h. 336 Cyclen durchlaufen.

Beispiel 4

Durchführung wie in Beispiel 1 jedoch unter Einsatz von 0,6 g (2,2 mmol) Bis(cyclooctadien) nickel (O) ($COD_2Ni$) und äquivalenten Mengen $HBF_4$ sowie $PMe_3$, Ni : AME = 1 : 1 000. Nach 8 Stunden betrug der Umsatz 11 % und die Selektivität, bezogen auf linearen Diester, 90 %.

Beispiel 5

0,39 g (2 mmol) $AgBF_4$ wurden in 10 ml $CH_2Cl_2$ suspendiert und bei — 30 °C mit einer Lösung von 0,42 g (2 mmol $\eta^3$-$C_3H_5NiCl(PMe_3)$ in 30 ml $CH_2Cl_2$ versetzt. Nach 90 Minuten wurden bei 0 °C 17 g (0,2 mol) AME (auf 0 °C vorgekühlt) zugesetzt (Ni : AME = 1 : 100)). Nach 10 Stunden wurde, wie in Beispiel 1 angegeben, aufgearbeitet und analysiert. Es ergab sich ein Umsatz von 83,5 % sowie eine Selektivität von 90 %, bezogen auf lineare Dimere. Die linearen Dimere setzten sich wie folgt zusammen : 93,8 % trans-Hexen-2-disäuredimethylester, 5,4 % cis-Hexen-2-disäuredimethylester, 1,3 % trans-Hexen-3-disäuredimethylester. Insgesamt wurden pro Nickelatom 42 Cyclen durchlaufen.

Beispiele 6-8

Arbeitsweise wie in Beispiel 5, jedoch wurden äquivalente Mengen folgender Phosphane statt $PMe_3$ eingesetzt : $P(n\text{-}C_4H_9)_3$ (Beispiel 6), $P(n\text{-}C_8H_{17})_3$ (Beispiel 7), $C_6H_5P(CH_3)_2$ (Beispiel 8).

| Beisp. | Phosphan | % Umsatz nach 10 h | Cyclen-zahl | % Selektivität bezogen auf lineare Dimere |
|---|---|---|---|---|
| 5 | $P(CH_3)_3$ | 83,5 | 42 | 90 |
| 6 | $P(n\text{-}C_4H_9)_3$ | 70,5 | 35 | 87 |
| 7 | $P(n\text{-}C_8H_{17})_3$ | 49,5 | 25 | 86 |
| 8 | $C_6H_5P(CH_3)_2$ | 48,1 | 25 | 85 |

Beispiele 9-10

Arbeitsweise wie in Beispiel 5, jedoch unter Verwendung äquivalenter Mengen von $AgPF_6$ (Beispiel 9) bzw. $AgSbF_6$ (Beispiel 10).

| Beisp. | Anion | % Umsatz nach 10 h | Cyclen-zahl | % Selektivität bezogen auf lineare Dimere |
|---|---|---|---|---|
| 5 | $BF_4$ | 83,5 | 42 | 90 |
| 9 | $PF_6$ | 46,3 | 23 | 91 |
| 10 | $SbF_6$ | 46,2 | 23 | 87 |

Beispiele 11-13

Arbeitsweise wie in Beispiel 5, jedoch unter Verwendung verschiedener Lösungsmittel :

| Beisp. | Lösungsmittel | % Umsatz nach 10 h | Cyclenzahl | % Selektivität bezogen auf lineare Dimere |
|---|---|---|---|---|
| 5 | $CH_2Cl_2$ | 83,5 | 42 | 90 |
| 11 | Chlorbenzol | 30,1 | 15 | 89 |
| 12 | Nitrobenzol | 50,8 | 25 | 88 |
| 13 | Toluol | 27 | 13 | 91 |

Beispiele 14-17

Arbeitsweise wie in Beispiel 5, jedoch Durchführung bei verschiedenen Temperaturen :

| Beisp. | Temperatur, °C | % Umsatz nach 10 h | Cyclenzahl | % Selektivität bezogen auf lineare Dimere |
|---|---|---|---|---|
| 14 | -20 | 8 | 15 | 87 |
| 15 | 0 | 8 | 41 | 90 |
| 16 | +20 | 2 | 29 | 87 |
| 17 | +20 | 6 | 33 | 89 |

Beispiele 18-20

Darstellung des Katalysators wie in Beispiel 1, Durchführung der Katalysen jedoch bei unterschiedlichen Temperaturen und Konzentrationen :

| Bsp. | Temp. °C | RZ h | $Ni:CH_2Cl_2$ mmol/ml | Ni:AME mmol/mmol | Cyclenzahl | % Selektivität |
|---|---|---|---|---|---|---|
| 18 | -10 | 312 | 1 / 176 | 1 : 1792 | 165 | 91 |
| 19 | 0 | 73 | 1 / 202 | 1 : 1082 | 383 | 90 |
| 20 | +10 | 120 | 1 / 430 | 1 : 2269 | 219 | 90 |

Beispiele 21-25

Arbeitsweise wie in Beispiel 5, jedoch Durchführung mit unterschiedlichen Konzentrationen an $CH_2Cl_2$ und AME ;
Reaktionsbedingungen : 8 h, RT

| Bsp. | $Ni:CH_2Cl_2$ mmol/ml | Ni:AME mmol/mmol | Cyclen-zahl | % Selektivität bezogen auf lineare Dimere |
|---|---|---|---|---|
| 21 | 1 / 7.1 | 1 : 507 | 17 | 85 |
| 22 | 1 / 75 | 1 : 314 | 100 | 85 |
| 23 | 1 / 104 | 1 : 404 | 111 | 91 |
| 24 | 1 / 219 | 1 : 371 | 126 | 88 |
| 25 | 1 / 440 | 1 : 590 | 144 | 96 |

Beispiele 26-28

Arbeitsweise wie in Beispiel 5, jedoch unter Verwendung von Methacrylsäuremethylester (MAME) anstelle von AME. Laut GC enthielt das Destillat neben nicht umgesetzen MAME 2,5-Dimethylhexen(2)-disäuredimethylester (2,5-Dime-2-HDM) als Hauptprodukt (der linearen und verzweigten Dimeren).

| Bsp. | Temp. | RZ h | Cyclen-zahl | % 2,5-Dime-2-HDM (bezogen auf umges. MAME) |
|---|---|---|---|---|
| 26 | 0°C | 10 | 4 | 87 |
| 27 | RT | 10 | 9 | 79 |
| 28 | RT | 20 | 13 | 83 |

Beispiel 29

0,44 g (2,07 mmol) $(\eta^3C_3H_5)NiCl(PMe_3)$ wurden in 50 ml Chlorbenzol mit 0,40 g (2,07 mmol) $AgBF_4$ 1,5 Stunden bei —30 °C gerührt. In die Katalysatorlösung ließ man 68,4 g (0,79 mol) Acrylsäuremethylester (AME) einfließen. (Ni : AME ≙ 1 : 385) und erwärmte auf 50 °C. Nach 6 Stunden wurden alle flüchtigen Produkte im Vakuum abgezogen. Laut Gaschromatographie betrug der Umsatz 17,03 %, d. h. 11,6 g AME wurden dimerisiert. Das Destillat enthielt neben 56,7 g AME 10,2 g lineare und 1,4 g verzweigte monomere Diester. Die Selektivität bezogen auf lineare Diester betrug 87,8 %. Pro mol Nickel wurden demnach 58 Mol AME dimerisiert bzw. 29 Cyclen durchlaufen.

**Patentansprüche**

1. Verfahren zur katalytischen Dimerisation von Derivaten der Acrylsäure in homogener Phase, dadurch gekennzeichnet, daß man Verbindungen der allgemeinen Formel $H_2C = CR^1—COOR^2$, in der $R^1$ für H oder lineare Alkylgruppen mit 1 bis 3 C-Atomen und $R^2$ für lineare Alkylgruppen mit 1 bis 3 C-Atomen stehen, in einem Lösungsmittel bei Temperaturen von —50 °C bis + 50 °C in Gegenwart von Nickelverbindungen als Katalysatoren umsetzt, die komplexe Anionen X in einem Molverhältnis Ni : X = 1 : 1 und organische oder Hybrid-Liganden enthalten und mit Phosphanen der allgemeinen Formel $PR_2R'$ modifiziert werden, in der R und R' gleich oder verschieden sein können und für Alkylreste mit 1 bis 8 C-Atomen oder Phenylreste stehen.

2. Verfahren nach Anspruch 1, dadurch gekennzeichnet, daß man als Monomere Acrylsäuremethylester oder Methacrylsäuremethylester einsetzt.

3. Verfahren gemäß Anspruch 1 und 2, dadurch gekennzeichnet, daß die löslichen Nickelverbindungen als weitere Anionen Alkyl-, Cycloalkyl-, Aralkyl-, Allyl-, Cycloalkenyl- oder Arylgruppen oder Hydridionen enthalten.

4. Verfahren gemäß Anspruch 1 bis 3, dadurch gekennzeichnet, daß als Katalysatoren lösliche Nickelverbindungen verwendet werden, die als komplexe Anionen X $BF_4$, $PF_6$ bzw. $SbF_6$-Anionen enthalten.

5. Verfahren gemäß Anspruch 1 bis 4, dadurch gekennzeichnet, daß zur Modifizierung der löslichen Nickelverbindungen Tri-n-alkylphosphane $PR_2R'$, in denen R und R' gleich sind und für lineare Alkylreste mit 1 bis 8 C-Atomen stehen, bevorzugt Trimethylphosphan, in einem Molverhältnis Ni : $PR_2R'$ = 1 : 0,5 bis 1,5 eingesetzt werden.

6. Verfahren gemäß Anspruch 1 bis 5, dadurch gekennzeichnet, daß die Dimerisation in Methylenchlorid durchgeführt wird.

7. Verfahren nach Anspruch 1 bis 6, dadurch gekennzeichnet, daß bei Temperaturen von — 20 °C bis + 20 °C gearbeitet wird.

8. Verwendung der entsprechend dem Verfahren gemäß Ansprüchen 1 bis 7 erhaltenen Dimeren als Monomere und/oder Comonomere in Polymerisations-, Polykondensations- und Hydrierverfahren.

**Claims**

1. A process for the catalytic dimerization of derivatives of acrylic acid in the homogeneous phase, characterized in that a compound having the general formula $H_2C = CR^1—COOR^2$, wherein $R^1$ represents H or a linear alkyl group having from 1 to 3 carbon atoms and $R^2$ represents a linear alkyl group having from 1 to 3 carbon atoms, is reacted in a solvent at a temperature of from — 50 °C to + 50 °C in the presence of nickel compounds as catalysts which contain complex anions X in a molar ratio Ni : X = 1 : 1 and organic or hydride ligands and are modified with phosphanes having the general formula $PR_2R'$, wherein R and R' may be same or different and represent alkyl residues having from 1 to 8 carbon atoms or phenyl residues.

2. The process according to claim 1, characterized in that acrylic acid methyl ester or methacrylic acid methyl ester are used as monomers.

3. The process according to claims 1 and 2, characterized in that the soluble nickel compounds contain alkyl, cycloalkyl, aralkyl, allyl, cycloalkenyl, or aryl groups or hydride ions as further anions.

4. The process according to any one of claims 1 to 3, characterized in that soluble nickel compounds containing $BF_4$, $PF_6$ or $SbF_6$ anions as complex anions X are used as the catalysts.

5. The process according to any one of claims 1 to 4, characterized in that tri-n-alkyl phosphanes $PR_2R'$, wherein R and R' are the same and represent linear alkyl residues having from 1 to 8 carbon atoms, and preferably trimethyl phosphane, in a molar ratio of Ni : $PR_2R'$ of from 1 : 0.5 to 1.5 are used for modifying the soluble nickel compounds.

6. The process according to any one of claims 1 to 5, characterized in that the dimerization is carried out in methylene chloride.

7. The process according to any one of claims 1 to 6, characterized in that temperatures of from — 20°C to + 20 °C are employed.

8. Use of the dimers prepared by the process according to any one of claims 1 to 7 as monomers and/or comonomers in polymerization, polycondensation and hydrogenation processes.

**Revendications**

1. Procédé de dimérisation catalytique de dérivés d'acide acrylique en phase homogène, caractérisé en ce que l'on fait réagir des composés de formule générale $H_2C = CR^1—COOR^2$ dans laquelle $R^1$ représente H ou des groupes alkyles linéaires avec 1 à 3 atomes de C et $R^2$ représente des groupes alkyles linéaires avec 1 à 3 atomes de C, dans un solvant à des températures de —50 °C à + 50 °C en présence de composés du nickel comme catalyseurs qui contiennent des anions complexes X dans un rapport molaire Ni : X = 1 : 1 et des ligands organiques ou à base d'hydrure et sont modifiés par des phosphanes de formule générale $PR_2R'$ dans laquelle R et R' peuvent être identiques ou différents et représentent des alkyles avec 1 à 8 atomes de C ou des phényles.

2. Procédé selon la revendication 1, caractérisé en ce qu'on met en œuvre comme monomères de l'acrylate de méthyle ou du méthacrylate de méthyle.

3. Procédé selon les revendications 1 et 2, caractérisé en ce que les composés de nickel solubles contiennent comme anions additionnels des groupes alkyles, cycloalkyles, aralkyles, allyles, cycloalcényles ou aryles ou des ions hydrure.

4. Procédé selon les revendications 1 à 3, caractérisé en ce que des composés de nickel solubles contenant $BF_4$, $PF_6$ ou $SbF_6$ en tant qu'anions complexes X sont employés comme catalyseurs.

5. Procédé selon les revendications 1 à 4, caractérisé en ce que, pour la modification des composés de nickel solubles, on met en œuvre des tri-n-alkylphosphanes $PR_2R'$ dans lesquels R et R' sont identiques et représentent des alkyles linéaires avec 1 à 8 atomes de C, de préférence du triméthylphosphane, dans un rapport molaire Ni : $PR_2R'$ = 1 : 0,5 à 1,5.

6. Procédé selon les revendications 1 à 5, caractérisé en ce que la dimérisation est conduite dans du chlorure de méthylène.

7. Procédé selon les revendications 1 à 6, caractérisé en ce qu'on opère à des températures de — 20 °C à + 20 °C.

8. Utilisation des dimères obtenus conformément au procédé selon les revendications 1 à 7 comme monomères et/ou comme comonomères dans des procédés de polymérisation, de polycondensation et d'hydrogénation.